# EUROPEAN PATENT SPECIFICATION

(11) **EP 3 829 611 B1**
(45) Date of publication and mention of the grant of the patent: **28.09.2022**
(21) Application number: 19758813.0
(22) Date of filing: 01.08.2019
(51) Int. Cl.: A61K 36/185, A61K 31/375, A61K 31/51, A61K 31/593, A61K 33/08, A61K 45/06, A61P 1/04, A61P 9/12, A23L 33/105, A23L 33/15, A23L 33/16

(54) **NUTRACEUTICAL FORMULATION FOR THE CONTROL OF ARTERIAL HYPERTENSION**
NUTRAZEUTISCHE FORMULIERUNG ZUR KONTROLLE DER ARTERIELLEN HYPERTONIE
FORMULATION NUTRACEUTIQUE POUR LA RÉGULATION DE L'HYPERTENSION ARTÉRIELLE

(30) Priority: 01.08.2018 IT 201800007747
(43) Date of publication of application: 09.06.2021
(73) Proprietor: Pharmanutrition R&D S.r.l., 20143 Milano MI (IT)
(72) Inventor: RISSA, Mauro, 20143 Milano MI (IT)
(74) Representative: Perani & Partners S.p.A.
(86) International application number: PCT/IB2019/056567
(87) International publication number: WO 2020/026187

(56) References cited:
- WO-A1-01/39601
- WO-A1-2017/040421
- US-B1- 8 303 995
- ARRIGO F. G. CICERO ET AL: "Food and plant bioactives for reducing cardiometabolic disease risk: an evidence based approach", FOOD & FUNCTION, vol. 8, no. 6, 1 January 2017 (2017-01-01) , pages 2076-2088, XP055580757, GB ISSN: 2042-6496, DOI: 10.1039/C7FO00178A cited in the application
- SCHNEIDER AARON C ET AL: "Reduced blood pressure responsiveness to skeletal muscle metaboreflex activation in older adults following inorganic nitrate supplementation", NITRIC OXIDE, vol. 78, 1 June 2018 (2018-06-01), pages 81-88, XP002790585, ISSN: 1089-8603 cited in the application
- NIKOLINA BANJANIN ET AL: "Changes of Blood Pressure and Hemodynamic Parameters after Oral Magnesium Supplementation in Patients with Essential Hypertension-An Intervention Study", NUTRIENTS, vol. 10, no. 5, 8 May 2018 (2018-05-08), page 581, XP055580746, DOI: 10.3390/nu10050581 cited in the application

## Description

### FIELD OF THE INVENTION

The present invention relates to a unitary oral composition, in particular in the form of nutraceutical, for use in the control of mild to moderate arterial hypertension, as defined in the claims.

### STATE OF THE ART

Hypertension is one of the most considered and potentially most subtle and risky cardiovascular risk factors in the context of cardiovascular diseases. Its control is therefore mainly pharmacological and occurs by changing a sedentary lifestyle and a diet rich in fats and sugars, so as to restore a condition of normal weight and better control of metabolic parameters and vascular physiology. However, there are a large number of subjects with signs of initial metabolic syndrome in the presence of a modest rise in both systolic and diastolic blood pressure, overweight and hyperglycaemia which, not yet candidates for antihypertensive pharmacological therapy with ACE inhibitors, sartans, diuretics or calcium - antagonists, can be well controlled with specific nutraceutical associations and with a correction of the lifestyle and of the eating habits.

In this context, it is particularly interesting the association of substances derived from the vegetable and mineral world favouring the normalization of systolic and diastolic pressure parameters, restoring vascular physiology without interfering, except to a negligible extent, with drugs and allowing a good patient management even for long periods, thus reducing the risk of serious cardiovascular events such as cardiac infarction and stroke. In particular, substances such as the aqueous and dry extract of *Beta Vulgaris* have shown a significant action in reducing blood pressure in patients with moderate hypertension. Other substances of nutraceutical use whose hypotensive action has been documented are ascorbic acid and magnesium, in the form of its organic and inorganic salts. However, the association of these substances in the same formulation is particularly complex, both for the liquid and solid forms, from the hygroscopy of the magnesium salts and of the ascorbic acid, from the reduced percentage of magnesium and of the same ascorbic acid and from the reduced percentage of magnesium of the most absorbable salts such as gluconate, pidolate, aspartate and bis-glycinate that makes it difficult to reach adequate dosages of magnesium per single tablet and capsule, as regards the solid forms, and from the poor stability of ascorbic acid in the form of aqueous solution (oxidation by oxygen and formation of dehydroascorbic acid) as well as from a difficult organoleptic correction of preparations for liquid forms. The main objective to design a nutraceutical capable of guaranteeing the maximum normalizing efficacy of peripheral vascular resistance should therefore be the combination of *Beta Vulgaris* extracts, magnesium salts and ascorbic acid in clinically effective dosages and in the form of tablets/capsules or granulates without of the stability problems just described above.

WO 2017/040421 A1 describes a composition for drastically raising NO levels and therefore reducing hypertension, comprising:
a) an actual amount of a source dependent on NO synthetase;
b) an effective amount of a source independent of NO synthetase;
c) an effective amount of a myeloperoxidase inhibitor.
ARRIGO F. G. CICERO ET AL: "Food and plant bioactives for reducing cardiometabolic disease risk: an evidence based approach", FOOD & FUNCTION, vol. 8, no. 6, 1 January 2017 (2017-01-01), pages 2076-2088, XP055580757, GB; ISSN: 2042-6496, DOI: 10.1039/C7F000178 A review all the bioactive substances contained in the food that turned out to be effective in preventing hypertension.

SCHNEIDER AARON C ET AL in "Reduced blood pressure responsiveness to skeletal muscle metaboreflex activation in older adults following inorganic nitrate supplementation", NITRIC OXIDE, vol. 78, 1 June 2018 (2018-06-01), pages 81-88, ISSN: 1089-8603 reports that *beta vulgaris* extract appears to be effective in preventing high blood pressure.

NIKOLINA BANJANIN ET AL: "Changes of Blood Pressure and Hemodynamic Parameters after Oral Magnesium Supplementation in Patients with Essential Hypertension - An Intervention Study", NUTRIENTS, vol. 10, no. 5, 8 May 2018 (2018-05-08), page 581, XP55580746, DOI: 10.3390/nu10050581 instead reports that magnesium oxide is effective in preventing hypertension.

### SUMMARY OF THE INVENTION

The Applicant has now surprisingly found that an association of *Beta Vulgaris* and the magnesium salt with ascorbic acid formed in situ, which has therefore a high bioavailability, is able to reduce both systolic and diastolic blood pressure values in hypertensive patients. In particular, this describes an association of *Beta Vulgaris* dry extract, magnesium oxide and ascorbic acid in the form of powders or granulates to be compressed or dispersed in water before intake or in the form of capsules.

The association mentioned is such that, once dispersed in acidic gastric fluid with a pH ranging from 1.5 to 2.5, it gives rise to the formation of a high-bioavailability magnesium ascorbate salt. The magnesium oxide in fact, once in contact with the gastric liquid, neutralizes its pH up to a value of 6.0 - 6.5, after the formation of magnesium hydroxide and the simultaneous formation of magnesium chloride according to the following reaction (I):

- (pH = 1.5-2.00) MgO + 2HCl = MgCl₂ + H₂O (pH = 6.0-6.5) (I)

Simultaneously, ascorbic acid at pH 6.0 - 6.5 is, in its predominant form, in the form of sodium salt, namely a salt that can react with magnesium chloride to give rise to magnesium ascorbate according to the following reaction (II):

- (pH = 6.0-6.5) MgCl₂ + 2 Na Asc = Mg (Asc)₂ + 2 NaCl (II)

The in situ formation of the magnesium ascorbate salt guarantees an excellent bioavailability of both magnesium and ascorbate at the enteric level. Surprisingly, the in vitro simulation of alkalization of the simulated fasting gastric fluid (Fasted state, 36 ml, pH = 1.5 for dilute hydrochloric acid and 0.9% saline solution) for one hour, maintained at 37°C and under stirring of 200 rpm with magnetic stirrer, on the part of the aforesaid composition in which the magnesium oxide was present in a minimum amount of 100 mg, maintained the pH of the gastric fluid between 5.5 and 6.0 even after the addition of aliquots of HCl diluted every 15 minutes, in order to simulate physiological gastric acid secretion. This experimental evidence supports the formation of the magnesium ascorbate salt and its permanence in the gastric lumen up to the discharge in the first tract of the small intestine where it will be absorbed. The advantages of the present composition with respect to the insertion of the already formed magnesium ascorbate salt in powder form in the tablet/capsule or granulate are the following:
- magnesium ascorbate is a very hygroscopic salt that can alter the physical and chemical stability of the pharmaceutical form, while the magnesium oxide and the non-salified ascorbic acid are much less hygroscopic substances, so that individually they are already effectively conveyed in these forms;
- the magnesium ascorbate salt in the present composition is formed in situ in a pseudo-neutral gastric environment due to the chemical mechanisms described above, which allow it to remain for the entire gastric tract up to the discharge in the first section of the small intestine where absorption will take place. In the case of the pre-prepared magnesium ascorbate salt, the acid pH of the stomach would convert it into ascorbic acid and magnesium chloride;
- the magnesium ascorbate salt that is formed in situ in the stomach according to the present composition can be conveniently taken by patients suffering from gastritis, gastric ulcer and/or gastro-oesophageal reflux, thanks to the concomitant alkalinisation of the gastric contents.

It is therefore the object of the present invention a unitary oral composition comprising as sole active ingredients
- *Beta vulgaris* dry extract,
- magnesium oxide,
- ascorbic acid and vitamins B1 and D3, for use in the control of mild to moderate arterial hypertension, in which:
   (i) ascorbic acid and magnesium oxide are in such weight ratios as to guarantee a molar ratio between ascorbic acid and magnesium from a minimum of 0.25:1 to a maximum of 1:2 and (ii) said unitary oral composition is in the form of tablets, water-dispersible granulates or powders, or capsules.

### DETAILED DESCRIPTION OF THE INVENTION

For the purposes of the present invention, the definition "unitary composition" means a single composition for example a tablet, a capsule, a single-dose sachet.

For the purposes of the present invention, the definition "comprising" followed for example by a list of components does not exclude the presence of further components besides those listed. On the contrary, the definition "constituted" followed for example by a list of components excludes the presence of further components besides those listed.

For the purposes of the present invention, the definitions of mild and moderate hypertension are those corresponding to the values indicated in the following Table 1 suggested by OMS/ISH.

**Table 1: Classification of suggested hypertension based on blood pressure levels in adults aged 18 and over**

| **Category** | ***Arterial pressure in mm Hg*** | |
|---|---|---|
| | **Systolic** | **Diastolic** |
| Optimal | < 120 | < 80 |
| Normal | < 130 | < 85 |
| Normal - high | 130 - 139 | 85 - 89 |
| Grade 1 hypertension - borderline | 140 - 149 | 90 - 94 |
| Grade 1 hypertension - mild | 150 - 159 | 95 - 99 |
| Grade 2 hypertension - moderate | 160 - 179 | 100 - 109 |
| Grade 3 hypertension - severe | ≥180 | ≥110 |
| Isolated systolic hypertension - borderline | 140 - 149 | < 90 |
| Isolated systolic hypertension | ≥150 | < 90 |

According to one aspect of the invention, it is preferable that magnesium oxide and ascorbic acid are present in the oral composition for use according to the present invention in a minimum amount of 250 mg.

It is clear that the maximum amount will be such as to provide the maximum daily dosage of ascorbic and magnesium acids as established by the Ministry of Health, which for the aforementioned assets is of 500 mg and 450 mg, respectively. According to another aspect of the invention, the *Beta vulgaris* extract in the oral composition for use according to the present invention is included in a dosage ranging from 250 to 750 mg and more preferably it is 500 mg.

According to the invention, the oral composition for use according to the present invention contains vitamin D3 and vitamin B1.

The presence of these vitamins in the compositions object of the present invention serves to implement the overall hypotensive effect of the present composition [Golzarand M et al. Effect of vitamin D3 supplementation on blood pressure in adults: An updated meta analysis. Nutr Metab Cardiovasc Dis. 2016 Aug; 26 (8): 663-73, França CF, Vianna LM. Effectiveness of B vitamins on the control of hypertension and stroke events of SHRSP rats. J Diet Suppl. 2010 Mar; 7 (1): 71-7]. The unitary oral compositions for use according to the present invention are in the form of tablets, or else they are water-dispersible granulates or powders and finally they can be in the form of capsules and, according to a particularly preferred solution they are in the form of water-dispersible powders or granulates in the form of single-dose sachets.

The unitary oral composition for use according to the present invention is preferably administered once a day before meals, in order to avoid excessive gastric acid secretion, minimize gastric transit time and the interaction of the preparation described herein with the components of the food bolus.

Preferably, the oral composition is a nutraceutical formulation.

For the purposes of the present invention, a nutraceutical is, in its original definition, a food, or part of a food with proven beneficial and protective effects on both the physical and psychological health of the individual according to what is reported in the review entitled NUTRACEUTICA: DEFINIZIONE, REGOLAMENTAZIONE AND APPLICAZIONI by A. Pirillo and A.L. Catapano and published in the Giornale Italiano di Farmacoeconomia e Farmacoutilizzazione 2014; 6 (4): 23-30. The unitary oral compositions for use according to the present invention comprise excipients suitable for stabilizing pharmaceutical forms such as silica, magnesium stearate, fillers, such as corn starch, maltodextrin, and polyols.

The following is an example of an oral composition for use according to the present invention in the form of a water-dispersible single-dose sachet.

### Example 1

| Raw material | Mg/sachet | Excess formula (mg) |
|---|---|---|
| Maltodextrin | 1199.887 | 0.000 |
| Protected vitamin C (protected ascorbic acid) | 512.821 | 0.000 |
| Magnesium oxide | 507.700 | 0.000 |
| Beet (Beta vulgaris) - roots dry extract 1:4 | 500.000 | 0.000 |
| Flavour | 150.000 | 0.000 |
| Coated vitamin B1 (thiamine mononitrate) 33.3% | 92.593 | 0.000 |
| silicon dioxide | 15.000 | 0.000 |
| Vitamin D3 100 (100,000 UI/G) | 10.000 | 2.000 |
| Acesulfame K | 5.000 | 0.000 |
| Sucralose | 5.000 | 0.000 |
| Total weight mg | 3.000 | |

The following is a clinical study carried out on mild or moderately hypertensive patients who were administered the oral composition precisely for use according to the present invention.

### Example 2 - CLINICAL STUDY

### 2.1. Premise

The international guidelines for the management of arterial hypertension and risk of cardiovascular disease suggest the improvement of lifestyle in the healthy way¹ as a first step in the treatment of patients suffering from pre-hypertension or hypertension of first degree. Indeed, there is no doubt that a correct dietary approach (especially when associated with a global lifestyle change) is associated with a significant reduction in cardiovascular risk in the general population and in the hypertensive patient². However, lifestyle changes are often not simple and give results only after a long time; this reduces, on the one hand, the compliance and persistence to changes, while on the other hand it reduces the expected effectiveness of these interventions.

A possible support to the modification of lifestyle can come from the use of some supplements or nutraceuticals with an antihypertensive action, which, when effective, can guarantee a significant reduction in blood pressure, even on top of standard therapy³. In fact, meta-analysis of placebo-randomized clinical trials have clearly demonstrated that different substances of natural origin can individually induce pressure drops of 2-8 mmHg of pressure and 2-5 mmHg of diastolic pressure⁴. Some of them require very high dosages (e.g. fatty acids omega 3), others are too expensive (e.g. lactotripeptides), while others are burdened with side effects (e.g. aged garlic extracts)⁵. On the other hand, other nutraceuticals such as donors of nitric oxide, magnesium and folic acid can determine an improvement in the endothelial function and consequently a decrease in blood pressure⁶.

In this context, it has been carried out a placebo-controlled pilot clinical trial to evaluate how the combination of evidence-based antihypertensive nutraceuticals could improve home pressure control in the short and medium term in subjects suffering from pre-hypertension or hypertension of first degree.

### 2.2. Materials and methods [page 9 of Italian text]

The study was carried out on 36 pre-hypertensive and hypertensive first-degree patients, not pharmacologically treated, with estimated moderate cardiovascular risk, according to the SCORE algorithm.

The study was carried out according to the ethical rules of the Helsinki declaration and all patients have formally consented to the study. After 4 weeks of a low-sodium Mediterranean stabilization diet, during which patients were instructed to choose the correct foods, avoid excesses and increase their daily physical activity, the same subjects were randomly assigned to take the oral formulation reported in Example 1 or a placebo indistinguishable in colour and taste, both in the form of a water-dispersible sachet every morning before breakfast.

The previous three days, patients were asked to measure their home pressure at 7.00 (before breakfast) and at 19 (before dinner), taking 3 consecutive measurements, as per guidelines. The considered pre-treatment and post-treatment parameters (systolic, diastolic, pulse and mean arterial pressure) were derived from the average of the data collected in the 3 morning and evening measurements of the 3 days preceding the visit.

Total cholesterolaemia was monitored in order to estimate any changes in the risk estimated with the SCORE algorithm, while creatininemia was used to assess any changes in eGFR (estimated by the CKD-EPI formula), as a safety parameter.

The data were compared with ANCOVA using the SPSS 21.0 statistical software and considering a significance threshold of 0.05.

### 2.3 Results

36 subjects of average age 55 ± 8 years, equally distributed between men and women, were enrolled. Diet compliance was monitored and assessed as good throughout the duration of the study in both treatment groups.

No patient complained of side effects while taking the active product and placebo. No significant changes in creatinine and eGFR were observed in either group. The overall acceptance of the products was good in both groups and treatment compliance was close to 100%.

The changes in the main efficacy parameters were summarized in the following tables 1 (placebo) and 2 (treatment with the formulation of example 1).

**Table 1 - Changes in hemodynamic, laboratory and cardiovascular risk parameters in the placebo group**

| N. 36 | **Baseline** | **8 weeks** | **16 weeks** |
|---|---|---|---|
| Morning PAS (mmHg) | 146±8 | 141±9^{∗} | 140±7^{∗} |
| Morning PAD (mmHg) | 91±3 | 89±4 | 89±6 |
| Morning PP (mmHg) | 55±4 | 52±4 | 55±3 |
| Morning PAM (mmHg) | 109±11 | 106±10 | 107±8 |
| | | | |
| Evening PAS (mmHg) | 148±7 | 146±9 | 145±9 |
| Evening PAD (mmHg) | 92±4 | 91±4 | 90±3 |
| Evening PP (mmHg) | 56±3 | 55±4 | 55±3 |
| Evening PAM (mmHg) | 111±11 | 110±9 | 109±11 |
| | | | |
| Total cholesterol (mg/dL) | 192±14 | 174±13^{∗} | 177±12^{∗} |
| eGFR (ml/min) | 82±7 | 83±6 | 82±8 |
| Estimated CV risk (%) | 5,1±0,8 | 4,7±0,9 | 4,7±1,0 |

| | | | |
|---|---|---|---|
| ^{∗}*P < 0.05 vs. baseline* PAS = Systolic Blood Pressure, PAD = Diastolic Blood Pressure, PP = Wrist Pressure, PAM = Mean Arterial Pressure, eGFR = Estimated glomerular filtration, CV = Cardiovascular | | | |

**Table 2 - Variations of hemodynamic, laboratory and cardiovascular risk parameters in the group treated with the combined nutraceutical**

| N. 36 | **Baseline** | **8 weeks** | **16 weeks** |
|---|---|---|---|
| Morning PAS (mmHg) | 147±8 | 138±7^{∗}° | 139±8^{∗}° |
| Morning PAD (mmHg) | 92±4 | 86±3^{∗}° | 85±4^{∗}° |
| Morning PP (mmHg) | 55±4 | 52±4^{∗} | 54±3^{∗} |
| Morning PAM (mmHg) | 109±10 | 106±9^{∗}° | 107±9^{∗}° |
| | | | |
| Evening PAS (mmHg) | 149±9 | 145±7^{∗}° | 144±8^{∗}° |
| Evening PAD (mmHg) | 91±5 | 89±5 | 88±3^{∗} |
| Evening PP (mmHg) | 58±5 | 56±6 | 56±5 |
| Evening PAM (mmHg) | 111±10 | 108±9 | 107±9 |
| | | | |
| Total cholesterol (mg/dL) | 188±16 | 167±14^{∗} | 166±13^{∗} |
| eGFR (ml/min) | 82±7 | 83±6 | 82±8 |
| Estimated CV risk (%) | 5,2±0,8 | 4,1±0.9 | 4,2±1.0 |

| | | | |
|---|---|---|---|
| ^{∗}*P <0.05 vs. baseline, °P<0.05 vs. placebo* PAS = Systolic Blood Pressure, PAD = Diastolic Arterial Pressure, PP = Wrist Pressure, PAM = Mean Arterial Pressure, eGFR = Estimated glomerular filtration, CV = Cardiovascular | | | |

In the placebo group, it was observed a minimal but significant reduction in morning systolic blood pressure compared to baseline, compatible with the effect of continuing the modification of the lifestyle prescribed at the screening visit (that can also be deduced from the slight but significant improvement in total cholesterol that showed a similar trend).

The group treated with nutraceutical instead showed a significant reduction of all morning blood pressure parameters and only evening systolic blood pressure, both versus the baseline and versus the placebo group. This resulted in a significant reduction in the estimated cardiovascular risk only in the group treated with nutraceuticals, both versus the baseline and versus the placebo group.

### 2.4. Conclusions

The guidelines for the management of dyslipidaemia have already identified, since about ten years, a means of controlling lipid levels in patients at lower risk in nutraceuticals and functional cholesterol-lowering foods⁷. Despite an equivalent (if not greater) amount of evidence in favour of antihypertensive supplements, this type of approach has not yet been clearly developed. Yet, the most recent American guidelines for the identification and management of the hypertensive patient have particularly stressed the identification of patients with pressure previously defined as "normal-high" and the optimization of blood pressure control in subjects close to "normal" values⁸. This type of approach will involve on the one hand the identification of a much higher number of hypertensive subjects and on the other hand a high number of subjects with non-optimized pressure⁹. Given that the same guidelines stress how this phenomenon should not necessarily be managed with an increase in the pharmacological load, then it becomes increasingly interesting to find alternatives that can make the patient more adherent to lifestyle modifications and that can make such changes more effective in reducing blood pressure levels without inducing adverse events. The moderate, but significant, antihypertensive effect observed in our study was in line with that expected from the content of bioactive principles with vasodilatory action present in the tested integrator.

These effects were maintained even after the first 4 weeks of treatment and confirm, especially for the effects on blood pressure, that the preliminary results are not due to simple volume changes and that they do not lead to adaptation/tachyphylaxis phenomena. The final result was a significant reduction in the estimated cardiovascular risk according to the SCORE algorithm of about one percentage point with an excellent tolerability profile. The attenuation of efficacy in the evening, compared to the post-recruitment morning measurements, is probably linked to the short half-life of the components of the active nutraceutical, which also guarantees the absence of improbable accumulation phenomena and allows the time of administration to be shifted according to need. Finally, the active components are suitable to association with other antihypertensive drugs and to perform extravascular functions of particular importance (see magnesium, vitamin D, etc.)¹⁰. The main limitations of this exploratory study were the reduced sample size and the short duration of the test, so it is not currently known whether an adaptation to the effect, and therefore a reduction in long-term efficacy, can be had. However, the persistence of the effect on 16 weeks of treatment should exclude phenomena of tachyphylaxis. Further studies on several subjects and of longer duration must be carried out. However, the efficacy and safety of use of the individual components included in the formulation tested in our study has already been amply demonstrated in numerous studies of longer duration and adequately powerful.

In conclusion, the tested composite nutraceutical reduces medium-term systolic and diastolic blood pressure, thus leading to a significant reduction in the estimated cardiovascular risk in a sample of patients with pre-hypertension or hypertension of first degree.

### Bibliography

¹ Piepoli MF, Hoes AW, Agewall S, Albus C, Brotons C, Catapano AL, Cooney MT, Corrà U, Cosyns B, Deaton C, Graham I, Hall MS, Hobbs FD, Løchen ML, Löllgen H, Marques-Vidal P, Perk J, Prescott E, Redon J, Richter DJ, Sattar N, Smulders Y, Tiberi M, Bart van der Worp H, van Dis I, Verschuren WM. 2016 European Guidelines on cardiovascular disease prevention in clinical practice: The Sixth Joint Task Force of the European Society of Cardiology and Other Societies on Cardiovascular Disease Prevention in Clinical Practice (constituted by representatives of 10 societies and by invited experts) Developed with the special contribution of the European Association for Cardiovascular Prevention & Rehabilitation (EACPR). Atherosclerosis. 2016; 252: 207-74.
² Schwingshackl L, Hoffmann G. Diet quality as assessed by the Healthy Eating Index, the Alternate Healthy Eating Index, the Dietary Approaches to Stop Hypertension score, and health outcomes: a systematic review and meta-analysis of cohort studies. J Acad Nutr Diet. 2015 May;1 15(5):780-800.e5. doi: 10.1016/j.jand.2014.12.009.
³ Cicero AF, Colletti A. Nutraceuticals and Blood Pressure Control: Results from Clinical Trials and Meta-Analyses. High Blood Press Cardiovasc Prev. 2015 Sep;22(3):203-13. doi: 10.1007/s40292-015-0081-8.
⁴ Sirtori CR, Arnoldi A, Cicero AF. Nutraceuticals for blood pressure control. Ann Med. 2015;47(6):447-56. doi: 10.3109/07853890.2015.1078905.
⁵ Borghi C, Cicero AF. Nutraceuticals with a clinically detectable blood pressure-lowering effect: a review of available randomized clinical trials and their meta-analyses. Br J Clin Pharmacol. 2017 Jan;83(1):163-171. doi: 10.1111/bcp.12902
⁶ Cicero AF, Borghi C. Evidence of clinically relevant efficacy for dietary supplements and nutraceuticals. Curr Hypertens Rep. 2013 Jun;15(3):260-7. doi: 10.1007/s11906-013-0333-8.
⁷ Patti AM, Toth PP, Giglio RV, Banach M, Noto M, Nikolic D, Montalto G, Rizzo M. Nutraceuticals as an Important Part of Combination Therapy in Dyslipidaemia. Curr. Pharm. Des. 2017; 23(17): 2496-2503. doi: 10.2174/1381612823666170317145851.
⁸ Whelton PK, Carey RM, Aronow WS, Casey DE Jr, Collins KJ, Dennison Himmelfarb C, DePalma SM, Gidding S, Jamerson KA, Jones DW, MacLaughlin EJ, ACC/AHA/AAPA/ABC/ACPM/AGS/APhA/ASH/ASPC/NMA/PCNA Guideline for the Prevention, Detection, Evaluation, and Management of High Blood Pressure in Adults: A Report of the American College of Cardiology/American Heart Association Task Force on Clinical Practice Guidelines. Hypertension. 2017 Nov 13. pii: HYP.0000000000000065. doi: 10.1161/HYP.0000000000000065
⁹ Muntner P, Ovbiagele B, Smith SC Jr, Spencer CC, Stafford RS, Taler SJ, Thomas RJ, Williams KA Sr, Williamson JD, Wright JT Jr. 2017 American College of Cardiology/American Heart Association High Blood Pressure Guideline. J Am Coll Cardiol. 2017 Nov 6. pii: S0735-1097(17)41474-4. doi: 10.1016/j.jacc.2017.10.073.
¹⁰ Cicero AFG, Fogacci F, Colletti A. Food and plant bioactives for reducing cardiometabolic disease risk: an evidence based approach. Food Funct. 2017 Jun 21;8(6):2076-2088. doi: 10.1039/c7fo00178a.

## Claims

1. Unitary oral composition comprising as the sole active ingredients:
• *Beta vulgaris* dry extract,
• magnesium oxide,
• ascorbic acid and vitamins B1 and D3
for use in the control of mild to moderate arterial hypertension,
wherein:
i) the weight ratios between ascorbic acid and magnesium oxide are such to guarantee a molar ratio between ascorbic acid and magnesium ranging from a minimum of 0.25:1 to a maximum of 1:2;
ii) said unitary oral composition is in the form of tablets, water-dispersible granulates or powders, or capsules.

2. The unitary oral composition for use according to claim 1, wherein both the magnesium oxide and the ascorbic acid are present in a minimum amount of 250 mg.

3. The unitary oral composition for use according to any one of the claims from 1 and 2, wherein the *Beta vulgaris* extract is comprised in a dosage ranging from 250 to 750 mg and more preferably 500 mg.

4. The unitary oral composition for use according to anyone of claims 1-3 in the form of powders or granules in water-dispersible single-dose sachets.

5. The unitary oral composition for use according to any one of the claims from 1 to 4, wherein it is administered before meals, preferably once a day.

6. The unitary oral composition for use according to any one of the claims from 1 to 5, to be administered to patients suffering from gastritis, gastric ulcer and/or gastro-oesophageal reflux.

## Patentansprüche

1. Einheitliche orale Zusammensetzung, enthaltend als die einzigen Wirkstoffe:
• *Beta vulgaris* Trockenextrakt,
• Magnesiumoxid,
• Ascorbinsäure und die Vitamine B1 und D3
zur Behandlung der leichten bis mittelschweren arteriellen Hypertonie,
wobei:
i) die Gewichtsverhältnisse zwischen Ascorbinsäure und Magnesiumoxid so sind, dass ein Molverhältnis zwischen Ascorbinsäure und Magnesium von mindestens 0,25:1 bis höchstens 1:2 gewährleistet ist;
ii) die einheitliche orale Zusammensetzung in Form von Tabletten, wasserdispergierbaren Granulaten oder Pulvern oder Kapseln vorliegt.

2. Einheitliche orale Zusammensetzung zur Verwendung nach Anspruch 1, wobei sowohl das Magnesiumoxid als auch die Ascorbinsäure in einer Mindestmenge von 250 mg vorhanden sind.

3. Einheitliche orale Zusammensetzung zur Verwendung nach einem der Ansprüche 1 und 2, wobei der *Beta vulgaris Extrakt* in einer Dosierung von 250 bis 750 mg und vorzugsweise 500 mg enthalten ist.

4. Einheitliche orale Zusammensetzung zur Verwendung nach einem der Ansprüche 1 bis 3 in Form von Pulvern oder Granulaten in wasserdispergierbaren Einzeldosisbeuteln.

5. Einheitliche orale Zusammensetzung zur Verwendung nach einem der Ansprüche 1 bis 4, wobei sie vor den Mahlzeiten, vorzugsweise einmal täglich, verabreicht wird.

6. Einheitliche orale Zusammensetzung zur Verwendung nach einem der Ansprüche 1 bis 5 zur Verabreichung an Patienten, die an Gastritis, Magengeschwür und/oder gastroösophagealem Reflux leiden.

## Revendications

1. Composition orale unitaire comprenant comme seuls ingrédients actifs :
• extrait sec de *Beta vulgaris,*
• oxyde de magnésium,
• acide ascorbique et vitamines B1 et D3
pour une utilisation dans le contrôle de l'hypertension artérielle légère à modérée,
dans laquelle :
i) les rapports pondéraux entre l'acide ascorbique et l'oxyde de magnésium sont tels qu'ils garantissent un rapport molaire entre l'acide ascorbique et le magnésium allant d'un minimum de 0,25:1 à un maximum de 1:2 ;
ii) ladite composition orale unitaire se présente sous la forme de comprimés, de granulés ou de poudres dispersibles dans l'eau, ou de capsules.

2. Composition orale unitaire pour une utilisation selon la revendication 1, dans laquelle l'oxyde de magnésium et l'acide ascorbique sont tous deux présents en une quantité minimale de 250 mg.

3. Composition orale unitaire pour une utilisation selon l'une quelconque des revendications de 1 à 2, dans laquelle l'extrait de *Beta vulgaris* est compris dans un dosage allant de 250 à 750 mg et plus préférentiellement 500 mg.

4. Composition orale unitaire pour une utilisation selon l'une quelconque des revendications 1-3 sous la forme de poudres ou de granulés dans des sachets unidoses dispersibles dans l'eau.

5. Composition unitaire orale pour une utilisation selon l'une quelconque des revendications de 1 à 4, dans laquelle elle est administrée avant les repas, de préférence une fois par jour.

6. Composition orale unitaire pour une utilisation selon l'une quelconque des revendications de 1 à 5, à administrer à des patients souffrant de gastrite, d'ulcère gastrique et/ou de reflux gastro-œsophagien.
